# EUROPEAN PATENT APPLICATION

(11) **EP 1 559 328 A1**
(43) Date of publication of application: **03.08.2005**
(21) Application number: 04030874.4
(22) Date of filing: 22.03.2002
(51) Int. Cl.: A23K 1/00, A23K 1/18

(54) **Fungal feeds for aquaculture and agriculture**

(30) Priority: 23.03.2001 US 277947 P
(62) Divisional of application: 02725276.6
(71) Applicant: Advanced Bionutrition Corporation, Columbia, MD 21045 (US)
(72) Inventor: Kyle, David.J., Catonsville, MD 21228 (US)
(74) Representative: Warcoin, Jacques

(57) **Abstract**

This invention is directed to the use of fungal cells which are used as feed components in aquaculture or agriculture, and which also contain exogenous peptides, proteins, and/or antibodies, which will convey resistance or immunity to viral or bacterial pathogens or otherwise improve the health and performance of the species consuming said fungal cells.

## Description

### BACKGROUND OF THE INVENTION

### Field of the Invention

This invention is directed to the use of microbial cells which are used as feed components in aquaculture or agriculture, and which also contain exogenous peptides, proteins, and/or antibodies, which will convey resistance or immunity to viral or bacterial pathogens or otherwise improve the health and performance of the species consuming said microbial cells. The microbial cells can be yeast, fungi, bacteria, or algae. The proteins and/or antibodies may be expressed inside the microbial cells by direct genetic modification of the microbe itself, or by the infection of the microbe with a virus that has been altered to express the protein of interest.

### Related Art

Certain plant products have been produced using specific genetic modification to express proteins and/or antibodies of therapeutic value. The group at the Boyce Thompson Institute at Cornell has been cloning viral coat protein into bananas and potatoes so that when ingested, this will be equivalent to delivering an oral vaccine. This concept has not been extended to microbes.

There are several plant biotech companies such as Meristem, Large Scale Biology, and Prodigene, which are now expressing certain human therapeutic proteins in the plants including antibodies.

Recombinant microbes including bacteria, yeast and fungi have been used to produce human therapeutic proteins. However, such recombinant microbes have not been used for agricultural purposes incorporating ingestion of the whole organism. In both the plant and microbial cases, the recombinant organism has simply been used as a factory, and the therapeutic protein is then isolated and purified prior to use.

Certain plant products have been produced which contain proteins and/or antibodies of therapeutic value by infecting the plant with a virus that expresses the protein of interest. Large Scale Biology has a series of patents protecting this technology but these patents do not disclose the use of the technology in microbes and certainly not algae.

Antibiotic doping is used routinely in the aquaculture setting. Typically, the pure or semipure antibiotics are added directly to the water column; however, crude fermentation broths, or crude preparations including cells, have not been used for any kind of therapeutic delivery system.

Production of amino acids such as lysine typically involves a genetically modified microorganism, which overproduces the amino acid of interest and excretes it into the fermentation medium. The wastestream from such a fermentation would include biomass containing the amino acid, and this wastestream product could be used as a crude delivery form of the small molecule nutritive amino acid.

### SUMMARY OF THE INVENTION

The present invention provides for a composition of matter (the feed) and the use of this feed for the delivery of a therapeutic dose of a bioactive peptide or protein.

In one embodiment, this invention provides an aquaculture feed containing microbial biomass comprising one or more proteins, antibodies, or a combination thereof, where the proteins and antibodies are non-native to the microbes of the biomass. Preferably, the microbes are selected from yeast, fungi, bacteria, algae, or combinations thereof. The microbes may be engineered to recombinantly express the proteins or antibodies recombinantly, or the microbes may be infected with viruses or plasmids, which express the recombinant proteins or antibodies.

In another embodiment, this invention provides a method of delivering therapeutic proteins to an animal comprising administering to an animal a feed comprising a microbe expressing a non-native therapeutic protein. This method is particularly suitable for the non-human animal subjected to intensive agricultural practices, or for fish or shellfish in aquaculture. Preferred microbes are algae. In a preferred mode, the therapeutic protein is a recombinant protein expressed by the microbe or the microbe is infected by a recombinant virus, which expresses the recombinant therapeutic or bioactive protein. Preferred therapeutic proteins include a protein which inhibits growth or replication of Vibrio species in vitro, or a protein which inhibits Taura Syndrome Virus (TSV) or White Spot Syndrome Virus (WSSV) infection in shrimp, or a recombinantly expressed antibody.

### DETAILED DESCRIPTION OF THE EMBODIMENTS

Microalgae (single cell alga, or phytoplankton) represent the largest, but most poorly understood, kingdom of microorganisms on the earth. Like plants are to terrestrial animals, the microalgae represent the natural nutritional base and primary source of all the phytonutrients in the aquatic food chain. As the primary link in the aquatic food chain, microalgae are the source of many more phytonutrients than simply DHA and ARA. Microalgae also represent a vast genetic resource comprising in excess of 80,000 different species. Yeast, fungi and bacteria are also in the direct food chain of fish, crustaceans and mollusks. However, only a very few of these microbes, perhaps less than 10 species, have been exploited for aquaculture feeds. These few species have been used primarily for historical reasons and ease of cultivation. They have not been chosen on the basis of any scientific evidence of superiority as nutritional or therapeutic supplements.

The marine environment is filled with bacteria and viruses that can attack fish and shellfish thereby devastating intensive farms very quickly. Bacteria and viruses can also attack single celled microalgae, so these organisms have evolved biochemical mechanisms to defend themselves from such attacks. Such mechanisms may involve the secretion of compounds that inhibit bacterial growth or viral attachment. Such compounds are called "prebiotics" and have effects similar to how cranberry juice can prevent bladder infections in humans. When nutritional, therapeutic or protective effects are delivered via the whole live organisms, such as *Lactobacillis* in yogurt, such products are referred to as "probiotics" and the organisms are "probionts". For the purposes of this invention, both types of action will be referred to as probiotic.

Several algal species exhibit antibiotic activity. This activity may be due to certain bioactive constituents in the membranes or cell walls, the protein or the carbohydrate of the positively testing species that inhibit bacterial growth (prebiotics or herein probiotics). Any standard screening technique used to identify antibiotic agents may be used to screen for algae having antibiotic activity, including incubating filter disks soaked in culture broth from the candidate algae on a lawn of the target pathogenic microbe (e.g., Vibrio species). This invention contemplates the use of these "friendly algae" in a probiotic fashion to control the growth of certain "pathogenic microorganisms" in a pond. However, the main aspect of this invention is directed to the use of recombinant microbes or virus infected microbes to deliver the bioactive protein of choice. The recombinant microbes or virus-infected microbes may be tested for antibiotic activity by standard antibiotic screening assays to confirm their activity.

Historically, only bacteria have been used in a probiotic fashion to alter a pond's ecology in order to eliminate or reduce the number of pathogenic bacteria. A problem with the bacterial probiotic approach is that the existing microbial ecology represents a massive buffer that is difficult to modulate with the introduction of relatively small numbers of alternative bacteria and the results to date have been unimpressive. Furthermore, even if the newly introduced bacteria do bloom, any large increase in bacterial levels in a pond can lower oxygen levels and cause harm to the fish or shrimp. The use of photosynthetic microalgae overcomes this problem as they actually increase oxygen levels. Microalgae have not been considered before as probiotics. Previous experience in the screening of extensive algal culture collections has indicated a number of algal species that exhibit antibacterial or bacteriostatic capabilities. Some of these activities may be anti-Vibrio activity. Such species would be candidates for a high value enrichment feed that delivers both nutritional and antibiotic capabilities. This invention provides an approach to disease control which may be the solution to an impending ecological disaster that will result from the present uncontrolled practice of dumping of toxic chemicals and antibiotics into the water systems to control these bacterial, fungal or viral pathogens.

One of the major disease control problems in shrimp aquaculture today is infection by certain viruses (e.g., White Spot Syndrome Virus and Tara Syndrome Virus). Neither current antibiotic, nor probiotic strategies will work in this situation, and shrimp cannot be vaccinated in a way similar to fish. Shrimp have only a rudimentary immune system so they are particularly susceptible to devastation by viral attacks. This invention provides a solution to this problem using a biological control method using microalgae as the vector to deliver anti-White Spot antibodies directly to the shrimp. These "Designer feeds" would be a normal part of the diet, but modified to deliver a therapeutic dose of antibody directly to the gut of the shrimp. This approach is known as "passive immunity" because the antibody remains outside the host organism and simply prevents infestation through the gut wall. The invention envisions the use of transgenic algae, yeast, fungi or bacteria to deliver the antibody to the virus. Such probiotics, as envisioned in the invention, do not have to replicate in the target organism for the desired effect to occur. Alternatively, the microbe itself may be infected with a virus that is engineered to produce the antibody of interest. Alternatively, the microbial source may deliver a portion of the virus (e.g. a coat protein or coat proteins) or fragment thereof in order to immunize the shrimp, other shellfish, finfish or other animals.

Antibodies to desired targets, such as White Spot Syndrome Virus or Taura Syndrome Virus, may be prepared by routine immunization and selection of monoclonal antibody producing hybridomas, or by screening viral or bacterial expression libraries of immunoglobulin genes and gene fragments. See "Current Protocols in Immunology," Coligan, et al., eds, Wiley Interscience, 1991, and periodic supplements. Nucleic acid sequences encoding the binding sites of the selected antibodies can be cloned using standard methods (see "Current Protocols in Molecular Biology." Ausubel, et al., eds., Wiley-Interscience, 1987, and periodic supplements), and antibodies may be expressed from recombinant microbes (including algae, see, e.g., U.S. Patent No. 6,027,900) or cloned into viruses that infect the desired microbes.

There are a number of bactericidal and bacteriostatic peptides, which will inhibit microbial growth and that include, but are not limited to cecropins, penaeidins, bactenecins, callinectins, myticins, tachyplesins, clavanins, misgurins, pleurocidins, parasins, histones, acidic proteins, and lysozymes. These peptides may be expressed in a microbial biomass such as algae, yeast, fungi or bacteria using recombinant methods as described above, and thus provided as a feed component to convey resistance to infestation.

The invention as contemplated herein, is described in the following examples, but its utility is not limited to the examples provided.

### EXAMPLES

**Example 1. Selection of Useful Microbial Sources for Feeds that Provide Disease Control**. Microalgal biomass samples, aqueous extracts, organic extracts and extracts from the growth medium after cultivation of the algae were concentrated and spotted on filter paper discs. Using sterile techniques, these discs were then placed on agar plates overlaid with a lawn of selected organisms including but not limited to gram-negative bacteria, gram-positive bacteria, antibiotic resistant bacteria, yeast, or fungi. After incubation for an appropriate length of time to allow growth of the lawn of test organism the samples were plates were observed for zones of clearing (non-growth) around the filter paper discs. Large zones of clearing indicate potent antibiotic activity; small zones of clearing indicate less potent antibiotic activity.

**Example 2. Incorporation of an antibody into an algal feed**. A particular viral or bacterial pathogen is chosen and used to prepare monoclonal antibodies using procedures well known to experts in this field. Gene(s) coding for this antibody or an appropriate antibody fragment (Fab or Fv) are isolated and amplified in the appropriate vector. The gene is spliced into a transformation vector suitable for a eukarvotic algae or a prokaryotic alga (e.g. *Synechocystis*)*,* or a yeast (e.g. *Saccharomyces*) or a fungus (e.g. *Mortierella*)*.* The transformation vector is chosen so that the antibody will be over expressed in the microbial cell biomass. This biomass is then used as a feed additive in such a way as to provide the antibody directly to the animal thus providing passive immunity.

**Example 3. Expression of a bactericidal protein in a microbial feed.** A bactericidal protein is chosen for the particular application. For example, proteins of the penaeidin class may be chosen for pathogenic control in shrimp. Penaeidins are members of a family of antimicrobial peptides isolated from crustaceans (e.g., Penaeid shrimp). Antimicrobial peptides may also come from insects and chelicerates and may include but are not limited to cecropins, peneaidins, bactenecins, callinectins, myticins, tachyplesins, clavanins, misgunins, pleurocidins, parasins, histones, acidic proteins, and lysozymes. The gene for the chosen protein or peptide is either isolated from the original source, an amplification source, or it can be made synthetically. The gene is then incorporated into a transformation vector suitable for a eukaryotic algae (e.g. *Chlorella*) or a prokaryotic alga (e.g. *Synechocystis*), or a yeast (e.g. *Saccharomyces*) or a fungus (e.g. *Mortierella*). The transformation vector is chosen so that the protein will be over expressed in the microbial cell biomass. This biomass is then used as a feed additive in such a way as to provide the bactericidal protein directly to the animal thus providing resistance to that particular pathogen.

**Example 4. Vaccination using Feeds**. An antigen characteristic to a particular pathogen is chosen as is required by the animal and circumstances. For example, a viral coat protein(s) or component thereof, or an infectious bacterial protein, or a component thereof is chosen. The gene coding for the protein(s) is isolated and incorporated into a vector suitable for use in the microorganism of choice. The transformation vector is chosen so that the protein(s) will be over expressed in the microbial cell biomass. This biomass is then used as a feed additive in such a way as to provide the viral or bacterial or fungal protein(s) directly to the animal thus stimulating an immunological response to that particular pathogen. The microbial component may enter the body of the animal in the digestive tract, or otherwise through contact in the air or water.

**Example 5. Vaccination using probiotic Feeds**. Probiotic bacteria such as Lactobacillus, Bacillus, Bifidobacterium, etc. provide beneficial effects by their presence as live organisms in the digestive track of an animal. As such they are constantly replicating and become a significant portion of the intestinal microflora and make an excellent continuous delivery mechanism for oral vaccines. Oral vaccines must deliver the antigen to a portion of the intestinal mucosa where it can interact with immunogenic tissues (eg., Peyers Patches) and stimulate an immunogenic response. An antigen characteristic to a particular pathogen is chosen as is required by the animal and circumstances. For example, a viral coat protein or component thereof, or an infectious bacterial protein, or a component thereof is chosen. The gene coding for the protein is isolated and incorporated into a vector suitable for use in the probiotic microorganism of choice. Other gut microfloral components not generally considered as probiotics, but which live in the intestine, such as coliforms (e.g. Escherichia coli) can also be used as a vector for producing the vaccine in situ.

The two viral coat proteins from salmon infectious pancreatic necrosis virus (IPNV) are isolated and inserted into a transformation vector selected for use in Lactobacillus using molecular biology methods that are well know in the state of the art. The recombinant Lactobacillus cells expressing the viral antigens as free proteins, excreted proteins, and/or virus like particles (assembled viruses with no nucleic acid) are then grown using conventional fermentation technology, harvested and processed into a form usable as a feed for salmon. This form may include, but is not limited to freeze drying, spray drying, fluid bed drying, microencapsulation, extrusion, or tableting. The recombinant Lactobacillus is then provided to the salmon as a feed, thereby delivering both the valuable probiotic as well as the vaccine. In this case, the vaccine is constantly produced as long as the recombinant Lactobacillus is present in the gut of the animal.

**Example 6. Delivery of active peptides or proteins using probiotic feeds**. The gene for an active antimicrobial peptide, such as, but not limited to, cecropins, peneaidins, bactenecins, callinectins, myticins, tachyplesins, clavanins, misgurins, pleurocidins, or parasins, or an antimicrobial protein such as histones, acidic proteins, or lysozymes is isolated and inserted into a transformation vector selected for use in *Lactobacillus* using molecular biology methods that are well know in the state of the art. The recombinant *Lactobacillus* cells expressing the free peptides or proteins, or excreted proteins, are then grown using conventional fermentation technology, harvested and processed into a form usable as a feed for an animal such as, but not limited to fish, crustaceans, livestock, etc. This form may include, but is not limited to freeze drying, spray drying, fluid bed drying, microencapsulation, extrusion, or tableting. The recombinant *Lactobacillus* is then provided to the animal as a feed, thereby delivering both the valuable probiotic as well as the antimicrobial compound. In this case, the antimicrobial compound is constantly produced as long as the recombinant *Lactobacillus* is present in the gut of the animal.

## Claims

1. Feed composition comprising microbial biomass comprising one or more proteins, peptides, antibodies, antibody fragments, or a combination thereof, which are non-native to the microbes of the biomass, wherein the microbes are fungi.

2. Feed composition according to claim 1, wherein the proteins, peptides, antibodies, antibody fragments, or combination thereof are expressed by a recombinant virus infective to said microbial biomass.

3. Feed composition according to claim 1, wherein the protein, peptide, antibody, antibody fragments, or combination thereof is chosen from cecropins, penaeidins, bactenecins, callinectins, myticins, tachyplesins, clavanins, misgurins, pleurocidins, parasins, histones, acidic proteins, and lysozymes.

4. Feed composition according to claim 1, wherein the fungus is *Mortierella.*

5. Feed composition according to claim 1 for an animal.

6. Feed composition according to claim 5, wherein the animal is a non-human animal and is raised in aquaculture.

7. Feed composition according to claim 6, wherein the non-human animal is chosen from a fish, a crustacean, and a mollusk.

8. Feed composition according to claim 5, wherein the animal is a non-human animal and is raised in agriculture.

9. Feed composition according to claim 5, wherein the animal is a human.

10. Feed composition according to any of claims 1 to 9 for use as a medicament.

11. Feed composition according to claim 10, wherein the composition inhibits the growth or replication of a pathogen.

12. Feed composition according to claim 11, wherein the pathogen is a bacterial, a fungal, or a viral pathogen.

13. Feed composition according to claim 12, wherein the viral pathogen is chosen from Infectious Pancreatic Necrosis Virus, Taura Syndrome Virus, and White Spot Syndrome Virus.

14. Feed composition according to claim 12, wherein the bacterial pathogen is *Vibrio.*

15. Feed composition according to any of claims 1 to 9 for use as a vaccine.

16. Feed composition according to claim 15, wherein the protein, peptide, antibody, antibody fragments, or combination thereof confers active or passive immunity upon the animal.
